# EUROPÄISCHE PATENTSCHRIFT

(11) **EP 1 620 016 B1**
(45) Veröffentlichungstag und Bekanntmachung des Hinweises auf die Patenterteilung: **15.08.2007**
(21) Anmeldenummer: 04724000.7
(22) Anmeldetag: 29.03.2004
(51) Int. Cl.: A61B 10/00, A61B 17/34, G01N 1/08

(54) **KOAXIALKAN LE MIT DICHTELEMENT**
COAXIAL CANNULA PROVIDED WITH A SEALING ELEMENT
CANULE COAXIALE AVEC ELEMENT D'ETANCHEITE

(30) Priorität: 29.03.2003 DE 20305093 U
(43) Veröffentlichungstag der Anmeldung: 01.02.2006
(73) Patentinhaber: C.R. BARD, INC., Murray Hill, NJ 07974 (US)
(72) Erfinder: HESKE, Norbert, F., 82288 Kottgeisering (DE); HESKE, Thomas, 82284 Grafrath (DE)
(74) Vertreter: Tomlinson, Edward James
(86) Internationale Anmeldenummer: PCT/EP2004/003327
(87) Internationale Veröffentlichungsnummer: WO 2004/086977

(56) Entgegenhaltungen:
- EP-A- 0 433 717
- DE-U- 20 204 362
- US-A1- 2002 045 840

## Beschreibung

Die Erfindung bezieht sich auf eine in ein Gewebe einsetzbare Koaxialkanüle, in die zur Entnahme von Gewebe eine Biopsienadeleinheit mit Probeentnahmeraum, sowie eine die Biopsienadel koaxial an der Außenwand umgebende längsverschiebliche Probeabtrenneinrichtung aufweist, und wobei die Koaxialkanüle am proximalen Ende ein Dichtelement aufweist, das den Raum zwischen Koaxialkanüleninnenwand und Außenwand der Probenabetrenneinrichtung umschließt.

Aus der DE GMS 202 09 525.8 ist eine in ein Gewebe einsatzbare Koaxialkanüle bekannt, in die eine Biopsienadeleinheit einsetzbar ist. Um das Entweichen von Flüssigkeit einerseits zu verhindern und andererseits den Aufbau von Vakuum in dem zu biopsierenden Gewebe zu ermöglichen, ist eine Dichtung zwischen Koaxialkanüleninnenwand und Außenwand der Biopsienadeleinheit vorgesehen. In der GMS 202 09 523.8 ist ausgeführt, dass die Dichtfunktion der Dichtung so beschaffen sein muss, dass sie einen Luftaustritt oder Lufteintritt, sowie den Austritt von Flüssigkeit verhindert.

DE 202 04 362 U offenbart eine in ein Gewebe einsetzbare Koaxialkanüle, in die zur Entnahme von Gewebe eine Biopsienadel mit Probeentnahmeraum mit oder ohne eine die Biopsienadel koaxial an der Außenwand umgebende Probeabtrenneinrichtung einsetzbar ist, wobei am proximalen Ende der Kanüle ein Dichtelement aufgesetzt ist, das den Raum zwischen Kanüleninnenwand und Außenwand der Biopsienadel, oder Außenwand der Schneidhülse abdichtet.

Eine derartige Dichtung hat in der Praxis zu Problemen geführt. Beim Einbringen der Biopsienadeleinheit in die Koaxialkanüle und beim anschließenden Positionieren der Nadeleinheit wird die in der Koaxialkanüle durch den Einschiebevorgang eingedrungene Luft verdichtet und es bilden sich Luftblasen, die dazu führen, dass die bei der Positionierung der Nadel gemachten Aufnahmen mittels Ultraschall oder MR gestört werden, so dass eine genaue Positionierung wegen der Lufteinschlüsse nicht möglich ist.

Aufgabe der Erfindung ist es, dieses Problem zu lösen.

Die Lösung der Aufgabe wird darin gesehen, dass das Dichtelement beim Einschieben der Nadeleinheit den Luftaustritt freigibt und nach der Positionierung der Nadeleinheit und Anlegen eines Vakuums im Biopsienadelinnenraum einen Lufteintritt verhindert.

Durch eine derartige Ausbildung der Dichtung kann einerseits die durch das Einschieben der Nadeleinheit komprimierte Luft austreten, so dass keine eingeschlossenen Luftblasen gebildet werden, die Ultraschall- bzw. MR-Bilder werden nicht beeinflusst bzw. gestört.

Die Verwendung eines entsprechend dimensionierten Schlauchs, der auf das proximale Ende des Koaxialrohres aufgeschoben wird, ist eine einfache, billige aber wirksame Ausbildung des Dichtelements. Dabei ist darauf zu achten, dass die Flexibilität des Schlauchs so beschaffen ist, dass der beim Einführen vorhandene Spalt zwischen Koaxialkanüleninnenwand und Außenwand der Nadeleinheit bei geringem Unterdruck durch den Ansaugeffekt sicher verschlossen wird. Dies wird insbesondere dadurch erzielt, dass das proximale Ende, z.B. die Innenkante des Schlauchs, gegen die Außenseite der Nadeleinheit angesaugt wird. Aus diesem Grunde ist das proximale Ende des Schlauchs vorteilhafterweise zur Nadeleinheit hin geringfügig abgebogen, so dass beim Anlegen des Vakuums der überstehende Teil des Schlauchstückes nach innen gezogen wird und an die Außenfläche der Nadeleinheit gedrückt wird. Wird das Vakuum, der Unterdruck, im Nadelhohlraum abgebaut, so wird die Dichtwirkung aufgehoben und durch die Elastizität des Schlauches erfolgt wieder die Öffnung eines Spalts.

Das Dichtelement kann aber auch Teil des Vakuum-Biopsiegerätes (z. B. gemäß der DE- GMS 202 04 363) sein, insbesondere wenn das Biopsiegerät mit einer Führungsrolle ausgestattet ist. In diesem Fall ist distalseits an der Führungsrolle ein Stopfen angeordnet, der mit einer entsprechenden Kupplungsbohrung in der Kappe der Koaxialkanüle dichtend in Eingriff gebracht wird. Sofern die Dichtelemente in das Gegenstück erst kurz vor dem Aufsetzen des Gerätes auf das Gegenstück in die Gegenkupplungsstücke eintreten, kann die Luft vorher austreten. Die Wirkung tritt also erst kurz vor dem Verschließen ein, so dass keine Luftblasen oder Lufteinschlüsse die Ultraschall- oder MR-Bilder stören.
Sofern, zur Reduktion der Eindringtiefe der Biopsienadeleinheit zwischen Führungsrolle und proximaler Aufsatzfläche der Koaxialkappe ein Zwischenstück verwendet wird, weist das Zwischenstück distal- und proximalseitig je ein Kupplungsstück auf, so dass der Stopfen der Führungsrolle einerseits dichtend eingesetzt werden kann, und andererseits das Zwischenstück in die Kupplungskappe dichtend eingesetzt werden kann. Wichtig ist, dass das Verschließen des Zwischenraums zwischen Nadeleinheit und Koaxialkanüle erst kurz vor dem endgültigen Positionieren der Nadeleinheit erfolgt, so dass die Luft entweichen kann und nicht komprimiert wird.

Nachfolgend sind Ausführungsbeispiele im Einzelnen beschrieben. Es zeigt:
Fig. 1) Explosionsdarstellung einer Koaxialkanüle mit Eindrückdorn
Fig. 2) Schnitt durch die Kappe einer Koaxialkanüle (Variante A; vergrößert)
Fig. 3) Schnitt durch die Kappe einer Koaxialkanüle im Zusammenwirken mit einer Führungsrolle am Biopsiegerät (Variante B; vergrößert)
Fig. 4) Schnitt durch die Koaxialkanülenkappe unter Verwendung eines Zwischenstücks und einer Führungsrolle am Vakuum-Biopsiegerät

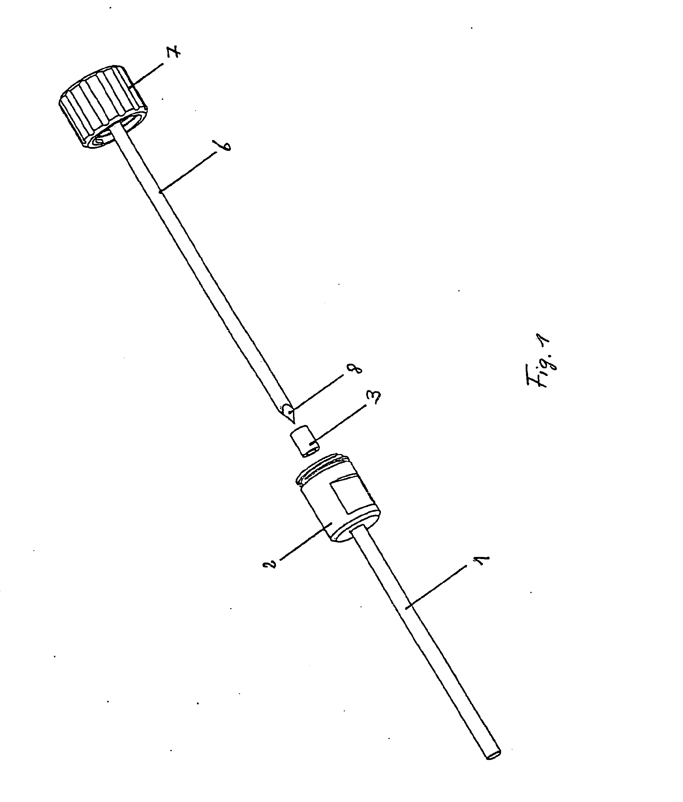

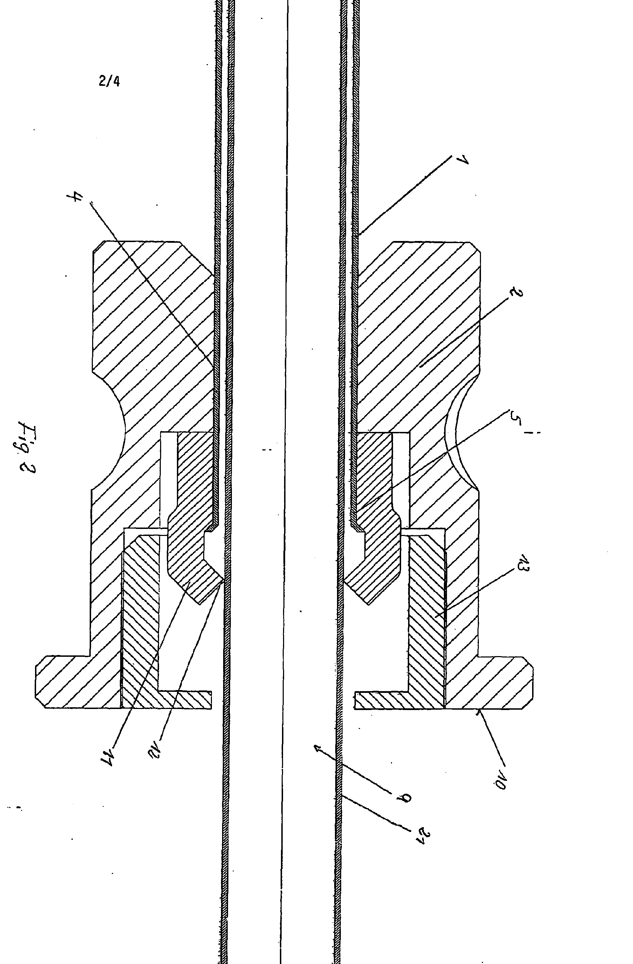

Fig. 1 zeigt eine Koaxialkanüle mit Eindrückdorn in einer Explosionsdarstellung. Mit dem Koaxialkanülenrohr 1 (kurz "Rohr" genannt) ist eine Kappe 2 verbunden. Zur Befestigung des Rohres 1 ragt dieses in eine Innenbohrung 4, der Kappe 2 mit seinem proximalen Ende 5 in die Kappe hinein (Fig. 2). Das proximale Rohrende 5 ist in der Kappe z.B. durch einen Klemmsitz gehalten. Auf das proximale Rohrende 5 wird ein Dichtelement 3 z.B. ein Schlauchstück aufgeschoben. Zum Einbringen der Koaxialkanüle in das Gewebe wird ein Dorn 6 (Eindrückdom) in die Koaxialkanüle eingeschoben und die Dornkappe 7 wird mit der Kappe 2 verschraubt. Die Dornspitze 8 steht im zusammengebauten Zustand über das distale Ende des Rohres vor.

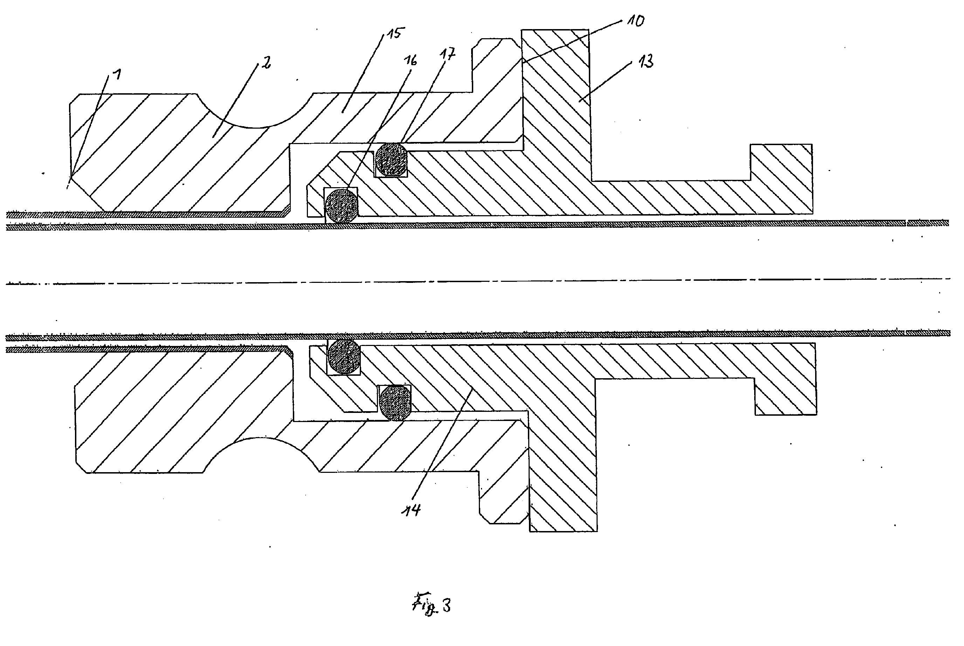

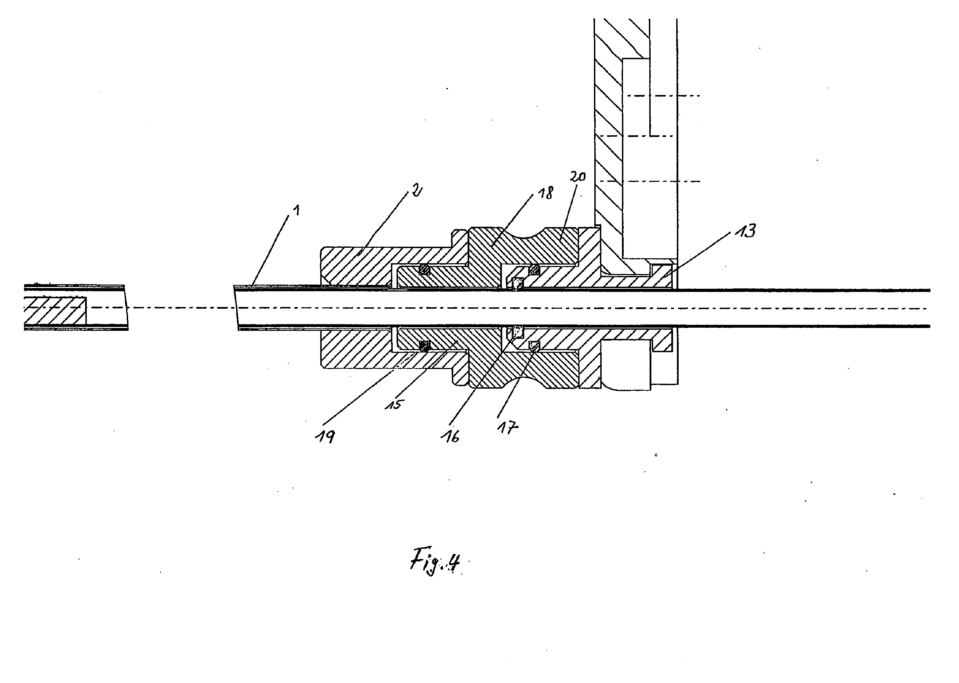

Die Koaxialkanüle wird zusammen mit dem Eindrückdorn in das Gewebe eingesetzt, z.B. durch Eindrücken, und zwar so, dass z.B. mittels eines Ultraschallgerätes die Dornspitze des Eindrückdorns nahe des zu untersuchenden Gewebes herangeführt, bzw. platziert wird. Nach dem Einsetzen der Koaxialkanüle mittels des Eindrückoms wird der Eindrückdorn durch Lösen z.B der Schraubverbindung zum proximalen Ende hin herausgezogen und entfernt. Um ein Verdrehen bzw. eine Veränderung der positionierten Koaxialkanüle zu verhindern sind an der Koaxialkanüle Flächen vorgesehen, in die eine Gabel oder Zange eingreift, die über weitere Elemente z.B. mit dem Operations- oder Untersuchungstisch verbunden sind, so dass die Koaxialkanüle in der einmal gewählten Position gehalten wird.
Nach dem Eindrücken und Positionieren der Koaxialkanüle wird nach Entfernen des Dorns die Nadeleinheit 9 eines Vakuum-Biopsiegerätes mit oder ohne außen angeordneter Schneidhülse 21 (Probenabtrenneinrichtung), in das Rohr 1 der Koaxialkanüle eingesetzt (Fig. 2). Die Nadeleinheit 9 besteht z.B. aus einer Hohlnadel mit einer diese koaxial umgebenden Schneidhülse 21, die distalseits eine Schneide trägt. Die Nadelvorrichtung kann aber ebenso eine außen liegende Hohlnadel sein, in deren Hohlraum koaxial die Abschneideinrichtung angeordnet ist. An Stelle der Domkappe sitzt nun die Stirnfläche z.B. einer sterilen Führungsrolle 13 des Vakuum-Biopsiegerätes auf der proximalen Stirnfläche 10 der Kappe 2 auf (sh. auch Fig. 3 und 4). Die Stirnfläche der Führungsrolle 13 des Vakuum-Biopsiegerätes sitzt nach dem Einsetzen auf der Stirnfläche 10 der Kappe auf. Die in die Koaxialkanüle nach Entfernen des Dorns eingedrungene Luft kann beim Einschieben der Nadeleinheit zunächst solange entweichen bis die Dichtlippe 11 durch ein angelegtes Vakuum in der Hohlnadel an die Außenfläche der Nadeleinheit herangezogen wird, d.h. das über das Kanülenrohr proximalseitig überstehende Teil des Dichtelements ist so ausgebildet, dass beim Einschieben der Nadeleinheit ein geringfügiger Spalt zwischen Dichtlippe und Außenfläche der Nadeleinheit offen bleibt; dies geschieht beispielsweise dadurch, dass nur eine Kante 12 der Dichtlippe die Außenfläche berührt. Bei Anlegen eines Vakuums im Hohlraum der Biopsienadel erhöht der Unterdruck die Anpresskraft, indem die Dichtlippe 11, also das freie Schlauchende an die Außenfläche der Nadeleinheit angedrückt wird und somit ein weiterer Lufteintritt verhindert wird.

Bei den in Fig. 3 und 4 dargestellten Beispielen, Alternativen für die Ausbildung der Abdichtfunktion, sitzt die Führungsrolle 13 nicht plan auf der Stirnfläche 10 der Kappe auf, und am proximalen Ende des Rohres 1 ist auch kein Dichtelement angeordnet, sondern die Führungsrolle des Biopsiegerätes weist einen Stopfen 14 auf, der in ein in der Kappe 2 vorgesehenes Kupplungsgegenstück 15 eingesetzt wird. Der Stopfen dichtet über die Dichtung 16 die Außenfläche der Nadeleinheit gegenüber der Führungsrolle ab. Mittels der Dichtung 17 wird die Kappe gegenüber dem Stopfen der Führungsrolle abgedichtet. Beide Dichtungen sind z.B. als O-Ringe ausgebildet. Da der Stopfen in Längserstreckung sehr kurz gehalten ist (z.B. Stopfenlänge 5 mm), erfolgt die Dichtwirkung erst kurz vor dem Aufsetzen der Führungsrolle auf die Kappe.
Die Dichtwirkung erfolgt also erst kurz vor der Positionierung der Nadelspitze. Bis zum Eintritt der Dichtwirkung kann die in der Koaxialkanüle vorhandene Luft entweichen.

Fig. 4 zeigt die gleiche Anordnung wie Fig. 3, jedoch wurde hier zur Verringerung der Eindringtiefe der Nadeleinheit ein Zwischenstück 18 zwischen Kappe 2 und Führungsrolle 13 eingefügt. Das Zwischenstück hat distalseits einen Stopfen, der in das Kupplungsgegenstück eingesetzt wird. Die Dichtung zwischen dem kappenseitigem Kupplungsgegenstück der Koaxialkanüle und dem Stopfen erfolgt über die Dichtung. Der Stopfen 14 der Führungsrolle entspricht dem bereits beschriebenen (Fig. 3) und wird in ein proximalseitig angeordnetes Kupplungsgegenstück 20 des Zwischenstücks 18 eingefügt. Die Dichtungsanordnung entspricht der bei Fig. 3 beschriebenen. Auch hier erfolgt die Dichtwirkung erst kurz bevor die Spitze der Nadeleinheit in ihre Endstellung gebracht wurde. Dadurch kann die eingedrungene Luft beim Einführungsvorgang aus der Hohlkanüle entweichen. Beide Lösungen zielen darauf ab, dass die beim Wechsel des Eindrückdorns gegen die Nadeleinheit die in die Koaxialkanüle eingedrungene Luft beim Einführen der Nadeleinheit möglichst vollständig entweichen kann, so dass keine Störungen beim Einsatz von Ultraschall oder MR auftreten.

### Teileliste i

1) Koaxialkanülenrohr (Rohr)
2) Kappe
3) Dichtelement
4) Innenbohrung
5) proximales Rohrende
6) Dorn
7) Dornkappe
8) Dornspitze
9) Nadeleinheit
10) Stirnfläche der Kappe
11) Dichtlippe
12) Kante
13) Führungsrolle
14) Stopfen
15) Kupplungsgegenstück
16) Dichtung
17) Dichtung
18) Zwischenstück
19) Dichtung
20) Kupplungsgegenstück
21) Schneidhülse (Probenabtrenneinrichtung)
22)
23)
24)
25)
26)

## Patentansprüche

1. In ein Gewebe einsetzbare Koaxialkanüle (1), die zur Entnahme von Gewebe eine Biopsienadeleinheit (9) mit Probeentnahmeraum sowie eine die Biopsienadel koaxial an der Außenwand umgebende, längsverschiebliche Probenabtrenneinrichtung (21) aufweist, wobei die Koaxialkanüle am proximalen Ende ein Dichtelement (3) aufweist, das den Raum zwischen Koaxialkanüleninnenwand und Außenwand der Probenabtrenneinrichtung umschließt, **dadurch gekennzeichnet, dass** das Dichtelement (3) beim Einschieben der Nadeleinheit den Luftaustritt freigibt, und dass nach dem Positionieren der Nadeleinheit und Anlegen eines Vakuums im Biopsienadelinnenraum einen Lufteintritt verhindert.

2. Koaxialkanüle nach Anspruch 1, **dadurch gekennzeichnet, dass** ein schlauchartiges Dichtelement (3) über das proximale Ende des Koaxialkanülenrohres (1) geschoben wird, dessen Innendurchmesser so bemessen ist, dass es einen geringen Spalt zwischen Dichtelement und Biopsienadeleinheit (9) frei lässt, und dass die Elastizität des Dichtelements (3) so bemessen ist, dass bei geringfügigem Unterdruck im Spalt zwischen Außenwand der Nadeleinheit (9) oder der Probenabtrenneinrichtung (21) und der Innenwand der Koaxialkanüle das proximale Ende des Dichtelements an der Nadeleinheit oder der Probenabtrenneinrichtung dichtend zur Anlage kommt.

3. In ein Gewebe einsetzbare Koaxialkanüle (1), die zur Entnahme von Gewebe eine Biopsienadeleinheit (9) mit Probeentnahmeraum sowie eine die Biopsienadel koaxial an der Außenwand umgebende, längsverschiebliche Probenabtrenneinrichtung (21) und eine Führungsrolle (13) aufweist, **dadurch gekennzeichnet, dass** an der distalen Fläche der Führungsrolle ein Stopfen (14) mit Dichtelementen (16,17) vorgesehen ist, der in ein Gegenstück (15) am proximalen Ende einer Kappe (2) der Koaxialkanüle eingefügt wird, so dass die Öffnung kurz vor dem Aufsetzen der distalen Fläche der Führungsrolle auf die proximale Fläche der Kappe der Koaxialkanüle verschlossen wird.

4. Koaxialkanüle nach Anspruch 3, **dadurch gekennzeichnet, dass** die Biopsienadeleinheit (9) zwischen der distalen Stirnfläche der Führungsrolle (13) mit Stopfen (14) und der proximalen Stirnfläche der Kappe (2) ein Zwischenstück aufweist, wobei das Zwischenstück proximalseitig ein Gegenkupplungsteil (20) in Art einer Innenbohrung aufweist, in die der Stopfen (14) der Führungsrolle (13) mit Dichtelementen eingesetzt wird und dass distalseitig das Zwischenstück (18) einen Stopfen mit Dichtelementen (19) aufweist, der in das proximalseitige Gegenstück (15) der Kappe (2) der Koaxialkanüle eingesetzt wird.

## Claims

1. A coaxial cannula (1) which is insertable into a tissue, which has, for removing tissue, a biopsy needle unit (9) with sample withdrawal space and also a longitudinally displaceable sample separating means (21) surrounding the biopsy needle coaxially to the outer wall, and the coaxial cannula having at the proximal end a sealing element (3) which surrounds the space between the inner wall of the coaxial cannula and the outer wall of the sample separating means, **characterised in that** the sealing element (3) opens the air outlet when the needle unit is inserted, and that after the needle unit has been positioned and a vacuum has been applied in the interior of the biopsy needle ingress of air is prevented.

2. A coaxial cannula according to Claim 1, **characterised in that** a tubular sealing element (3) is pushed over the proximal end of the coaxial cannula tube (1), the internal diameter of which is such that it leaves free a slight gap between the sealing element and the biopsy needle unit (9), and that the elasticity of the sealing element (3) is such that if there is a slight partial vacuum in the gap between the outer wall of the needle unit (9) or of the sample separating means (21) and the inner wall of the coaxial cannula the proximal end of the sealing element comes to lie hermetically against the needle unit or the sample separating means.

3. A coaxial cannula (1) which is insertable into a tissue, which has, for removing tissue, a biopsy needle unit (9) with sample withdrawal space and also a longitudinally displaceable sample separating means (21) and a guide roller (13) surrounding the biopsy needle coaxially to the outer wall, **characterised in that** a stopper (14) with sealing elements (16, 17) is provided on the distal surface of the guide roller, which stopper is inserted into a counter-piece (15) on the proximal end of a cap (2) of the coaxial cannula, so that the opening is closed shortly before the distal surface of the guide roller is placed on the proximal surface of the cap of the coaxial cannula.

4. A coaxial cannula according to Claim 3, **characterised in that** the biopsy needle unit (9) has an intermediate piece between the distal end face of the guide roller (13) with stopper (14) and the proximal end face of the cap (2), the intermediate piece on the proximal side having a counter-coupling part (20) in the manner of an internal bore into which the stopper (14) of the guide roller (13) is inserted with sealing elements, and that on the distal side the intermediate piece (18) has a stopper with sealing elements (19) which is inserted into the proximal-side counter-piece (15) of the cap (2) of the coaxial cannula.

## Revendications

1. Canule coaxiale (1) pouvant être insérée dans un tissu qui comprend une unité d'aiguille à biopsie (9) pour prélever des tissus avec un espace de prélèvement d'échantillon ainsi qu'un dispositif de séparation d'échantillon (21) pouvant être déplacé de manière longitudinale entourant de manière coaxiale l'aiguille à biopsie sur la paroi externe, la canule coaxiale comprenant à son extrémité proximale un élément d'étanchéité (3) qui entoure l'espace entre la paroi interne de la canule coaxiale et la paroi externe du dispositif de séparation d'échantillon, **caractérisée en ce que** l'élément d'étanchéité (3) libère la sortie d'air lors de l'insertion de l'unité d'aiguille, et **en ce qu'**après le positionnement de l'unité d'aiguille et la création d'un vide dans l'espace interne d'aiguille à biopsie il empêche une entrée d'air.

2. Canule coaxiale selon la revendication 1, **caractérisée en ce qu'**un élément d'étanchéité (3) tubulaire est poussé sur l'extrémité proximale du tube de la canule coaxiale (1) dont le diamètre intérieur est dimensionné de telle sorte qu'il laisse un interstice libre restreint entre un élément d'étanchéité et l'unité d'aiguille à biopsie (9), et **en ce que** l'élasticité de l'élément d'étanchéité (3) est dimensionnée de telle sorte qu'en cas de légère dépression dans l'interstice entre la paroi externe de l'unité d'aiguille (9) ou le dispositif de séparation d'échantillon (21) et la paroi interne de la canule coaxiale, l'extrémité proximale de l'élément d'étanchéité prend appui de manière étanche sur l'unité d'aiguille ou le dispositif de séparation d'échantillon.

3. Canule coaxiale (1) pouvant être insérée dans un tissu qui, pour prélever des tissus, comprend une unité d'aiguille à biopsie (9) avec un espace de prélèvement d'échantillon ainsi qu'un dispositif de séparation d'échantillon (21) pouvant être déplacé de manière longitudinale entourant l'aiguille à biopsie de manière coaxiale sur la paroi externe et un point de guidage (13), **caractérisée en ce qu'**un bouchon (14) avec des éléments d'étanchéité (16, 17) est prévu sur la surface distale du cylindre de guidage, lequel bouchon est inséré dans un élément complémentaire (15) à l'extrémité proximale d'un capuchon (2) de la canule coaxiale, de sorte que l'ouverture est obturée peu avant de placer la surface distale du cylindre de guidage sur la surface proximale du capuchon de la canule coaxiale.

4. Canule coaxiale selon la revendication 3, **caractérisée en ce que** l'unité d'aiguille à biopsie (9) entre la surface de contact distale du cylindre de guidage (13) avec des bouchons (14) et la surface de contact proximale du capuchon (2) comprend un élément intermédiaire, l'élément intermédiaire comprenant sur le côté proximal un élément d'accouplement complémentaire du type alésage interne, dans lequel le bouchon (14) du cylindre de guidage (13) est inséré avec des éléments d'étanchéité et **en ce que**, sur le côté distal, l'élément intermédiaire (18) comprend un bouchon avec des éléments d'étanchéité (19) qui est inséré dans l'élément complémentaire (15) du côté proximal du capuchon (2) de la canule coaxiale
